# EUROPÄISCHE PATENTSCHRIFT

(11) **EP 1 417 212 B1**
(45) Veröffentlichungstag und Bekanntmachung des Hinweises auf die Patenterteilung: **05.03.2008**
(21) Anmeldenummer: 02769970.1
(22) Anmeldetag: 07.08.2002
(51) Int. Cl.: C07F 9/6574, C07F 15/00, B01J 31/22, C07C 45/50

(54) **NEUE PHOSPHITVERBINDUNGEN UND DEREN Rh-KOMPLEXE**
NOVEL PHOSPHITE COMPOUNDS AND Rh-COMPLEXES THEREOF
NOUVEAUX COMPOSES DE PHOSPHITE ET LEURS COMPLEXES AVEC Rh

(30) Priorität: 16.08.2001 DE 10140083
(43) Veröffentlichungstag der Anmeldung: 12.05.2004
(73) Patentinhaber: Evonik Oxeno GmbH, 45772 Marl (DE)
(72) Erfinder: SCHMUTZLER, Reinhard, 38304 Wolfenbüttel (DE); NEDA, Ion, 38102 Braunschweig (DE); KUNZE, Christine, 38533 Vordorf (DE); BÖRNER, Armin, 18059 Rostock (DE); SELENT, Detlef, 10318 Berlin (DE); BORGMANN, Cornelia, 45657 Recklinghausen (DE); HESS, Dieter, 45770 Marl (DE); WIESE, Klaus-Diether, 45721 Haltern (DE)
(74) Vertreter: Hirsch, Hans-Ludwig
(86) Internationale Anmeldenummer: PCT/EP2002/008798
(87) Internationale Veröffentlichungsnummer: WO 2003/016320

(56) Entgegenhaltungen:
- EP-A- 1 201 675
- SIEDENTOP T. ET AL.: "Synthese und Komplexierung phosphorylierte spacer-modifizierter Glucosederivate" ZEITSCHRIFT FUR NATURFORSCHUNG, TEIL B: ANORGANISCHE CHEMIE, ORGANISCHE CHEMIE., Bd. 55, Nr. 10, - Oktober 2000 (2000-10) Seiten 956-960, XP002223607 VERLAG DER ZEITSCHRIFT FUR NATURFORSCHUNG. TUBINGEN., DE ISSN: 0932-0776
- SELENT D ET AL: "NEW PHOSPHORUS LIGANDS FOR THE RHODIUM-CATALYZED ISOMERIZATION/ HYDROFORMYLATION OF INTERNAL OCTENES" ANGEWANDTE CHEMIE. INTERNATIONAL EDITION, VERLAG CHEMIE. WEINHEIM, DE, Bd. 40, Nr. 9, 4. Mai 2001 (2001-05-04), Seiten 1696-1698, XP001009251 ISSN: 0570-0833
- KUNZE C ET AL: "CALIX(4)ARENE-BASED BIS-PHOSPHONITES, BIS-PHOSPHITES, AND BIS-O-ACYL-PHOSPHITES AS LIGANDS IN THE RHODIUM(I)-CATALYZED HYDROFORMYLATION OF 1-OCTENE" ZEITSCHRIFT FUER ANORGANISCHE UND ALLGEMEINE CHEMIE, VERLAG VON LEOPOLD VOSS, LEIPZIG, DE, Bd. 628, Nr. 4, 2002, Seiten 779-787, XP008010119 ISSN: 0044-2313

## Beschreibung

Die vorliegende Erfindung betrifft Phosphite und deren Metallkomplexe, die Herstellung, sowie die Verwendung der Phosphite als Liganden in katalytischen Reaktionen.

Die Reaktionen zwischen Olefinverbindungen, Kohlenmonoxid und Wasserstoff in Gegenwart eines Katalysators zu den um ein C-Atom reicheren Aldehyden ist als Hydroformylierung (Oxierung) bekannt. Als Katalysatoren in diesen Reaktionen werden häufig Verbindungen der Übergangsmetalle der 8. bis 10. Gruppe des Periodensystems der Elemente verwendet, insbesondere Verbindungen des Rhodiums und des Kobalts. Die Hydroformylierung mit Rhodiumverbindungen bietet im Vergleich zur Katalyse mit Kobaltverbindungen in der Regel den Vorteil höherer Selektivität und ist damit meistens wirtschaftlicher. Bei der durch Rhodium katalysierten Hydroformylierung werden zumeist Komplexe eingesetzt, die aus Rhodium und bevorzugt aus trivalenten Phosphorverbindungen als Liganden bestehen. Bekannte Liganden sind beispielsweise Verbindungen aus den Klassen der Phosphine, Phosphite und Phosphonite. Eine Übersicht über Hydroformylierung von Olefinen findet sich in B. CORNILS, W. A. HERRMANN, "Applied Homogeneous. Catalysis with Organometallic Compounds", Vol. 1&2, VCH, Weinheim, New York, 1996.

Jedes Katalysatorsystem (Kobalt oder Rhodium) hat seine spezifischen Vorzüge. Je nach Einsatzstoff und Zielprodukt werden unterschiedliche Katalysatorsysteme verwendet. Arbeitet man mit Rhodium und Triphenylphosphin, lassen sich α-Olefine bei niedrigeren Drücken hydroformylieren. Als phosphorhaltiger Ligand wird in der Regel Triphenylphosphin im Überschuss verwendet, wobei ein hohes Ligand/Rhodium-Verhältnis erforderlich ist, um die Selektivität der Reaktion zum kommerziell erwünschten n-Aldehydprodukt zu erhöhen.

US-A-4,694,109 und US-A-4,879,416 betreffen Bisphosphinliganden und ihren Einsatz in der Hydroformylierung von Olefinen bei niedrigen Synthesegasdrücken. Besonders bei der Hydroformylierung von Propen werden mit Liganden dieses Typs hohe Aktivitäten und hohe n/i-Selektivitäten erreicht.

In WO-A-95/30680 werden zweizähnige Phosphinliganden und ihr Einsatz in der Katalyse, unter anderem auch in Hydroformylierungsreaktionen, beschrieben.

Ferrocenverbrückte Bisphosphine werden beispielsweise in US-A-4,169,561, US-A-4,201,714 und US-A-4,193,943 als Liganden für Hydroformylierungen offenbart.

Selent D. et al, Angewandte Chemie, International Edition, Band 40, Nr. 9, WILEY-VCH Verlag GmbH, Weinheim 2001, Seiten 1696 bis 1698 offenbart Biphosphitliganden, die eine Phosphorinon-Struktureinheit aufweisen, und deren Verwendung für Hydroformylierungskatalysatoren.

Der Nachteil von zweizähnigen Phosphinliganden ist die relativ aufwendige Herstellung. Daher ist es oftmals nicht rentabel, solche Systeme in technischen Prozessen einzusetzen.

Rhodium-Monophosphit-Komplexe sind geeignete Katalysatoren für die Hydroformylierung von verzweigten Olefinen mit innenständigen Doppelbindungen, jedoch ist die Selektivität für endständig hydroformylierte Verbindungen gering. Aus EP-A-0 155 508 ist die Verwendung von bisarylensubstituierten Monophosphiten bei der rhodiumkatalysierten Hydroformylierung von sterisch gehinderten Olefinen, z. B. Isobuten bekannt.

Rhodium-Bisphosphit-Komplexe katalysieren die Hydroformylierung von linearen Olefinen mit end- und innenständigen Doppelbindungen, wobei überwiegend endständig hydroformylierte Produkte entstehen, dagegen werden verzweigte Olefine mit innenständigen Doppelbindungen nur in geringem Maße umgesetzt. Diese Phosphite ergeben bei ihrer Koordination an ein Übergangsmetallzentrum Katalysatoren von gesteigerter Aktivität, doch ist das Standzeitverhalten dieser Katalysatorsysteme, unter anderem wegen der Hydrolyseempfindlichkeit der Phosphitliganden, unbefriedigend. Durch den Einsatz von substituierten Bisaryldiolen als Edukte für die Phosphitliganden, wie in EP-A-0 214 622 oder EP-A-0 472 071 beschrieben, konnten erhebliche Verbesserungen erreicht werden.

Der Literatur zufolge sind die Rhodiumkomplexe dieser Liganden äußerst aktive Hydroformylierungskatalysatoren für α-Olefine. In US-A-4,668,651, US-A-4,748,261 und US-A-4,885,401 werden Polyphosphitliganden beschrieben, mit denen α-Olefine, aber auch 2-Buten mit hoher Selektivität zu den terminal hydroformylierten Produkten umgesetzt werden können, In US-A-5,312,996 werden zweizähnige Liganden dieses Typs auch zur Hydroformylierung von Butadien eingesetzt.

In EP 1 201 675 werden Biphosphitverbindungen beschrieben, die bis auf die Verbindung der Formel 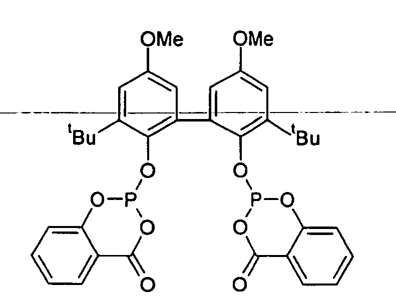 jeweils eine Phosphitstruktureinheit mit einer Acylgruppe und eine Phosphitstruktureinheit ohne Acylgruppe aufweisen. Diese Verbindungen werden als Liganden in Biphosphitmetallkomplexen verwendet, die in der Hydroformylierung eingesetzt werden.
Obgleich die genannten Bisphosphite gute Komplexliganden für Rhodium-Hydroformylierungskatalysatoren sind, ist es wünschenswert, neuartige leicht herstellbare Phosphite zur weiteren Verbesserung ihrer Wirksamkeit beispielsweise in der Hydroformylierung zu entwickeln.
Es wurde überraschend gefunden, dass neue Phosphite der allgemeinen Strukturformel I oder II, 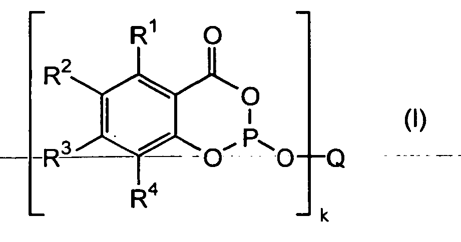 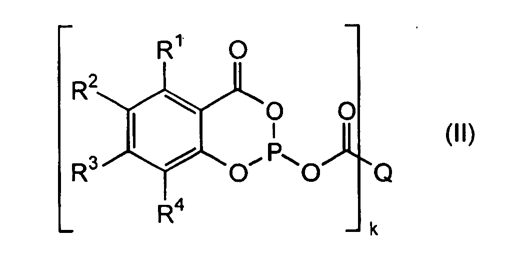 mit Ausnahme der Verbindung 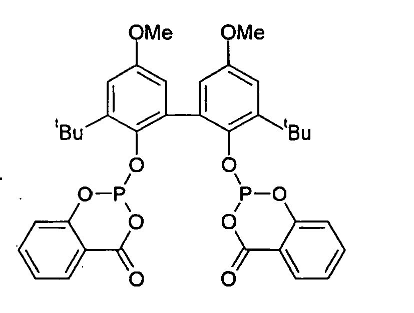 wobei R¹, R², R³, und R⁴ jeweils unabhängig voneinander ausgewählt sind aus einwertigen substituierten oder unsubstiuierten aliphatischen, alicyclischen, aromatischen, heteroaromatischen, gemischt aliphatisch-alicyclischen, gemischt aliphatisch-aromatischen, heterocyclischen, gemischt aliphatisch-heterocyclischen Kohlenwasserstoffresten mit 1 bis 50 Kohlenstoffatomen, H, F, Cl, Br, I, -CF₃, -CH₂(CF₂)ⱼCF₃ mit j = 0 - 9, -OR⁹, -COR⁹, -CO₂R⁹, -CO₂M, -SR⁹, -SO₂R⁹, -SOR⁹, -SO₃R⁹, -SO₃M, -SO₂NR⁹R¹⁰, -NR⁹R¹⁰, -N=CR⁹R¹⁰, wobei R⁹ und R¹⁰ unabhängig voneinander aus H, einwertigen substituierten oder unsubstituierten aliphatischen und aromatischen Kohlenwasserstoffresten mit 1 bis 25 Kohlenstoffatomen ausgewählt sind und M ein Alkalimetall-, formal ein halbes Erdalkalimetall-, Ammonium- oder Phosphoniumion ist,
oder benachbarte Reste R¹ bis R⁴ zusammen ein kondensiertes substituiertes oder unsubstituiertes aromatisches, heteroaromatisches, aliphatisches, gemischt aromatisch-aliphatisches oder gemischt heteroaromatisch-aliphatisches Ringsystem ausbilden; k mindestens 2 ist;
Q ein zweibindiger Kohlenwasserstoffrest gemäß Formel III 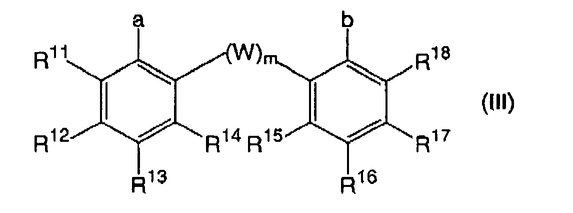 ist und R¹¹, R¹², R¹³, R¹⁴, R¹⁵, R¹⁶, R¹⁷ und R¹⁸ wie R¹ bis R⁴ definiert sind, W ein zweiwertiger Rest CR¹⁹R²⁰, wobei R¹⁹ und R²⁰ wie R⁹ und R¹⁰ definiert sind, m = 0 - 1 und
die Positionen a und b die Anknüpfpunkte darstellen, oder
Q ein k-bindiger Calix[n]arenrest gemäß Formel IV 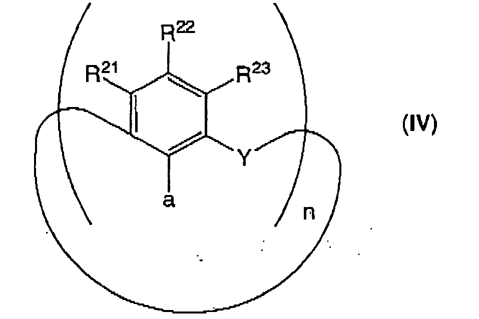 ist, wobei R²¹, R²², und R²³ unabhängig voneinander ausgewählt sind aus substituierten oder unsubstituierten aliphatischen und aromatischen Kohlenwasserstoffresten mit 1 bis 25 Kohlenstoffatomen, H, F, Cl, Br, I, -CF₃, -CH₂(CF₂)ⱼCF₃ mit j = 0-9, -OR²⁴, -COR²⁴, -CO₂R²⁴, -CO₂M, -SR²⁴, -SO₂R²⁴, -SOR²⁴, -SO₃R²⁴, -SO₃M, -SO₂NR²⁴R²⁵, -NR²⁴R²⁵, -N=CR²⁴R²⁵, wobei R²⁴ und R²⁵ wie R⁹ und R¹⁰ definiert sind und M ein Alkalimetall-, formal ein halbes Erdalkalimetall-, Ammonium- oder Phosphoniumion ist, oder benachbarte Reste R²¹ bis R²³ zusammen ein kondensiertes substituiertes oder unsubstituiertes aromatisches, heteroaromatisches, aliphatisches, gemischt aromatisch-aliphatisches oder gemischt heteroaromatisch-aliphatisches Ringsystem ausbilden;
Y ein zweiwertiger Rest CR²⁴R²⁵ oder CR²⁴R²⁵-O-CR²⁴R²⁵ ist, n = 3 - 6 ist und die Positionen a die Anknüpfpunkte darstellen oder für OR²⁴ stehen, wobei R²⁴ und R²⁵ wie oben definiert sind.

Gegenstand der Erfindung sind auch Komplexe der Phosphitliganden mit Rhodium und deren Herstellung.
Weiterhin ist die vorliegende Erfindung auf die Verwendung der Phosphite bzw. der Phosphitmetallkomplexe in der Hydroformylierung von Olefinen gerichtet.

Weitere Aspekte der Erfindung sind ein Verfahren zur Hydroformylierung von Olefinen sowie ein Verfahren zur Herstellung der Phosphitliganden.

In bevorzugten Phosphiten gemäß Formel I oder II bilden mindestens zwei benachbarte Reste R¹ bis R⁴ zusammen ein kondensiertes aromatisches, heteroaromatisches, aliphatisches, gemischt aromatisch-aliphatisches oder gemischt heteroaromatisch-aliphatisches Ringsystem, das unsubstituiert ist oder mit mindestens einem Rest, ausgewählt aus aliphatischen, alicyclischen, aromatischen, heteroaromatischen, gemischt aliphatisch-alicyclischen, gemischt aliphatisch-aromatischen, heterocyclischen, gemischt aliphatisch-heteroeyclischexi Kohlenwasserstoffresten mit 1 bis 50 Kohlenstoffatomen, F, Cl, Br, I, -CF₃, -CH₂(CF₂)ⱼCF₃ mit j = 0 - 9, -OR⁹, -COR⁹, -CO₂R⁹, -CO₂M, -SR⁹, -SO₂R⁹, -SOR⁹, -SO₃R⁹, -SO₃M, -SO₂NR⁹R¹⁰, -NR⁹R¹⁰ oder -N=CR⁹R¹⁰, substituiert ist, wobei R⁹, R¹⁰ und M wie zuvor definiert sind.

Repräsentative Phosphitliganden gemäß der Formel I oder II sind:

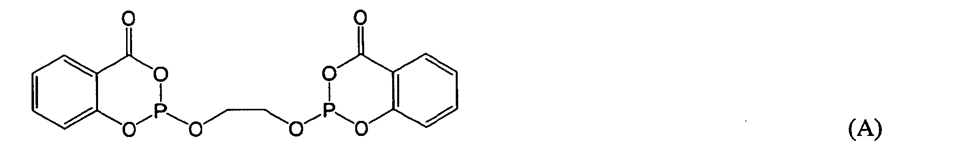

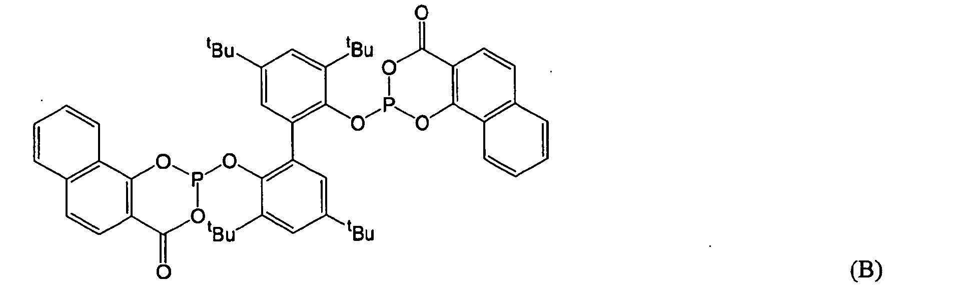

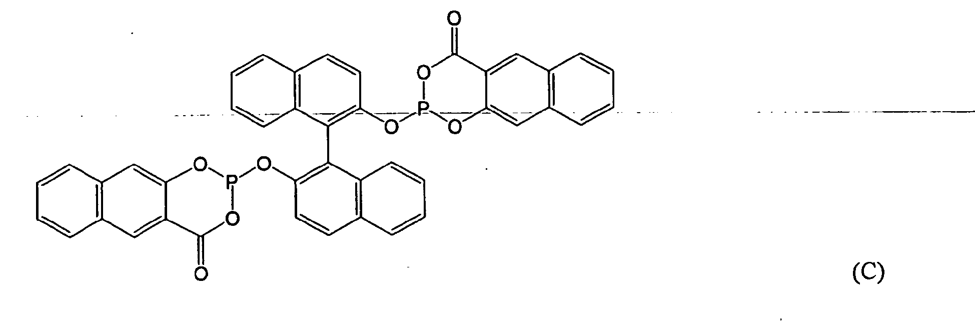

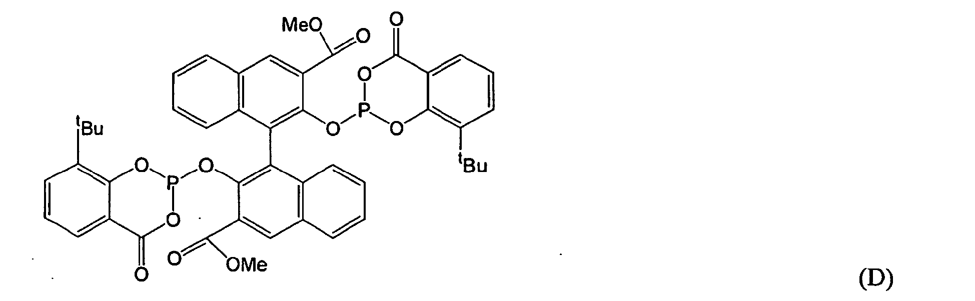

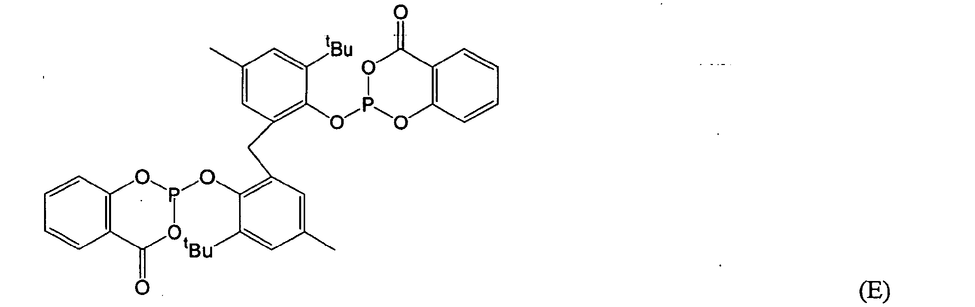

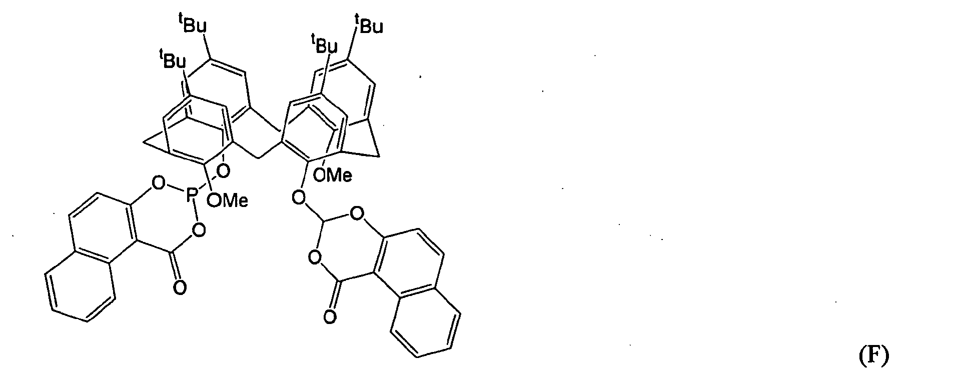

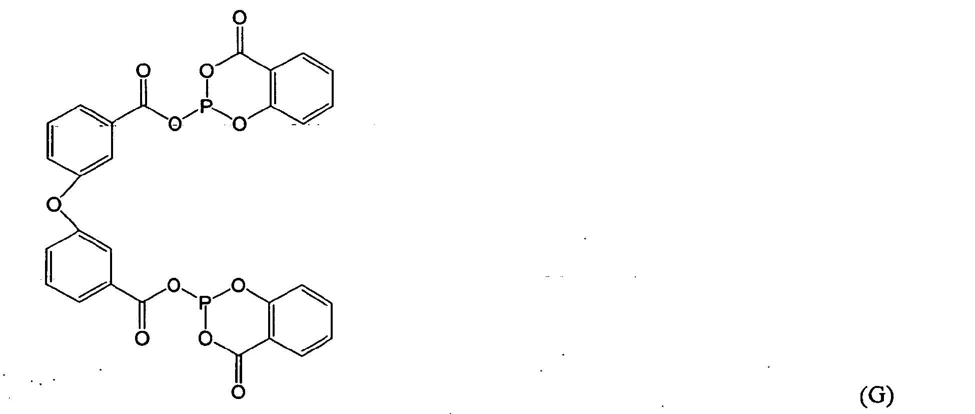

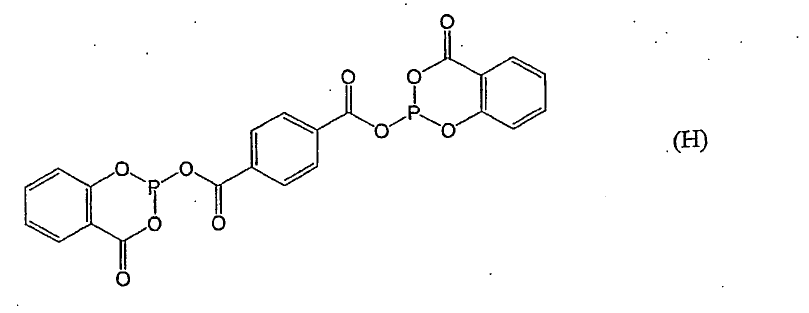

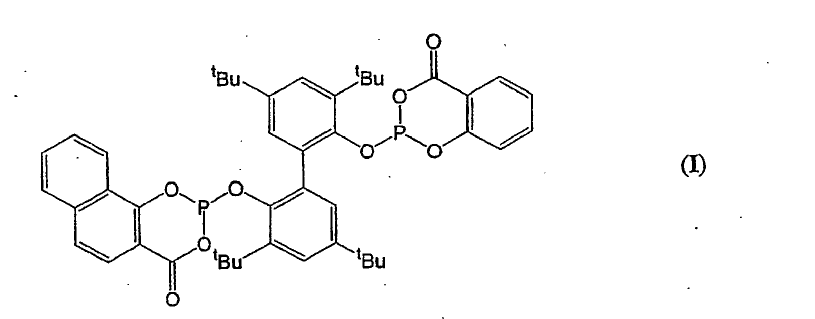

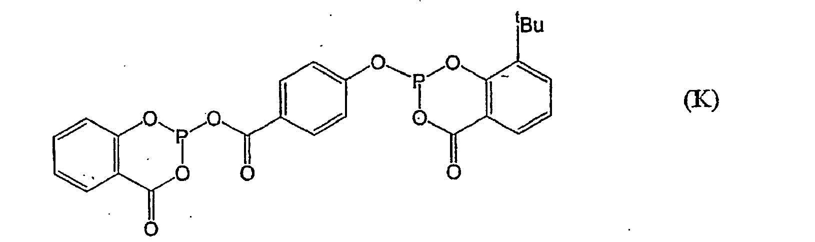

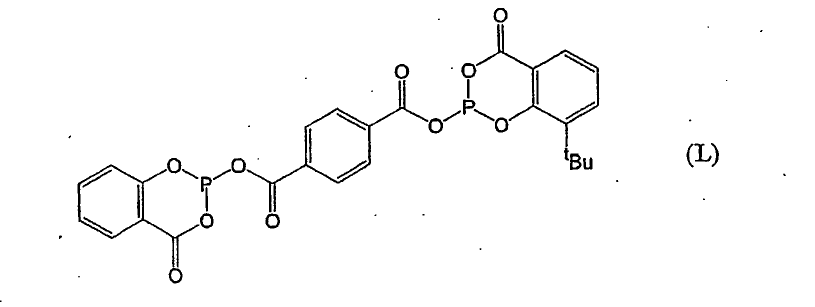

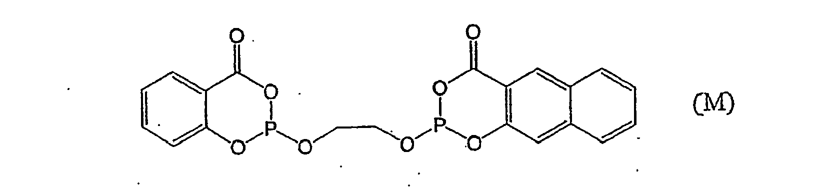

Bei den Beispielen (1) bis (M) handelt es sich um unsymmetrische Phosphite.

Die Phosphite zur erfindungsgemäßen Verwendung können durch eine Folge von Reaktionen von Phosphorhalogeniden mit Alkoholen, Carbonsäuren und/oder α-Hydroxyarylcarbonsäuren, bei denen Halogenatome am Phosphor gegen Sauerstoffgruppen ausgetauscht werden, hergestellt werden. Eine von mehreren Möglichkeiten zur Herstellung der erfindungsgemäßen Phosphite ist der folgende Syntheseweg für Bisphosphite:

In einem ersten Schritt wird eine α-Hydroxyarylcarbonsäure (1) mit einem Phosphortrihalogenid PX₃, wie etwa PCl₃, PBr₃ und PJ₃, vorzugsweise Phosphortrichlorid PCl₃, in Gegenwart einer Base, die vorzugsweise in äquivalenten oder in katalytischen Mengen eingesetzt wird, zu einem Halogendioxaphosphorinon (2) umgesetzt.

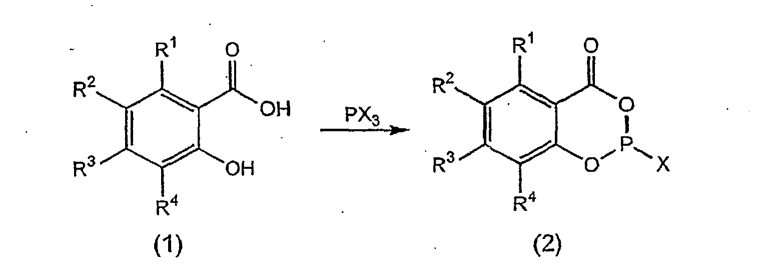

In einem zweiten Reaktionsschritt wird aus dem. Halogendioxaphosphorinon (2) durch Reaktion mit einem Diol (HO-Q-OH) bzw. einer Dicarbonsäure (HOOC-Q-COOH) in Gegenwart einer Base, die vorzugsweise in äquivalenten oder in katalytischen Mengen eingesetzt wird, das gewünschte Phosphit (I) bzw. (II) erhalten.

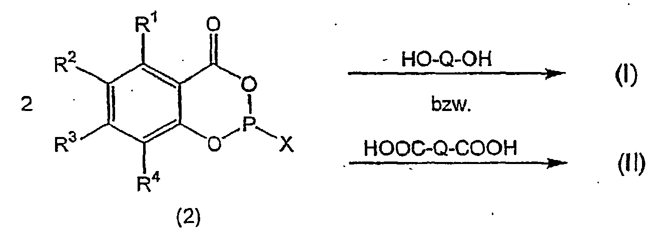

Die Reste R¹ bis R⁴ und Q haben die zuvor angegebenen Bedeutungen.

Bei der Synthese von unsymmetrischen Bisphosphiten werden im ersten Reaktionsschritt zwei unterschiedlich substituierte Chlordioxaphosphorinone (2a) und (2b) hergestellt, bei denen mindestens ein Rest R¹ bis R⁴ in (2a) und (2b) unterschiedliche Bedeutung hat und die dann im zweiten Reaktionsschritt nacheinander mit dem Diol bzw. der Dicarbonsäure in Gegenwart einer Base, die vorzugsweise in äquivalenten oder in katalytischen Mengen eingesetzt wird, umgesetzt werden.
Da die eingesetzten Diole bzw. Dicarbonsäuren und ihre Folgeprodukte häufig fest sind, werden die Umsetzungen im Allgemeinen in Lösungsmitteln durchgeführt. Als Solventien werden nichtprotische Lösungsmittel, die weder mit den Diolen bzw. Dicarbonsäuren noch mit den Phosphorverbindungen reagieren, verwendet. Geeignete Lösungsmittel sind beispielsweise Tetrahydrofuran, Ether wie Diethylether oder MTBE (Methyl-tertiärbutylether) oder aromatische Kohlenwasserstoffe wie Toluol.

Bei der Umsetzung von Phosphorhalogeniden mit Alkoholen entsteht Halogenwasserstoff, der durch zugegebene Basen gebunden wird. Beispielsweise werden dafür tertiäre Amine, wie Triethylamin, Pyridin oder N-Methylpyrrolidinon, eingesetzt. Teilweise ist es auch sinnvoll, die Alkohole vor der Reaktion in Metallalkoholate zu überführen, zum Beispiel durch Reaktion mit Natriumhydrid oder Butyllithium.

Die Phosphite sind geeignete Liganden zur Komplexierung von Metallen der 4., 5., 6., 7., 8., 9. oder 10. Gruppe des Periodensystems der Elemente. Die Komplexe können ein oder mehrere Phosphitliganden und gegebenenfalls weitere Liganden enthalten und sind als Katalysatoren geeignet, vorzugsweise bei der homogenen Katalyse. Beispiele für geeignete Metalle sind Rhodium, Kobalt, Iridium, Nickel, Palladium, Platin, Eisen, Ruthenium, Osmium, Chrom, Molybdän und Wolfram. Insbesondere mit Metallen der 8., 9. oder 10. Gruppe können die resultierenden Komplexe als Katalysatoren für Hydroformylierungs-, Carbonylierungs-, Hydrierungs- und Hydrocyanierungsreaktionen verwendet werden, besonders bevorzugt sind Rhodium, Kobalt, Nickel, Platin und Ruthenium. Beispielsweise ergeben sich in Hydroformylierungsreaktionen besonders bei Einsatz von Rhodium als Katalysatormetall hohe katalytische Aktivitäten. Die Katalysatormetalle kommen in Form von Salzen oder Komplexen zum Einsatz, im Falle von Rhodium z.B. Rhodiumcarbonyle, Rhodiumnitrat, Rhodiumchlorid, Rh(CO)₂(acac) (acac = Acetylacetonat), Rhodiumacetat, Rhodiumoctanoat oder Rhodiumnonanoat.

Aus den erfindungsgemäßen Phosphitliganden und dem Katalysatormetall bildet sich unter Reaktionsbedingungen die aktive Katalysatorspezies für die homogene Katalyse, etwa bei der Hydroformylierung bei Kontakt mit Synthesegas ein Carbonylhydridophosphit-Komplex. Die Phosphite und gegebenenfalls weitere Liganden können in freier Form zusammen mit dem Katalysatormetall (als Salz oder Komplex) in die Reaktionsmischung gegeben werden, um die aktive Katalysatorspezies in situ zu erzeugen. Es ist weiterhin auch möglich, einen Phosphitmetallkomplex, der die o..g. Phosphitliganden und das Katalysatormetall enthält, als Precursor für den eigentlichen katalytisch aktiven Komplex einzusetzen. Diese Phosphitmetallkomplexe werden hergestellt, indem man das entsprechende Katalysatormetall der 4. bis 10. Gruppe in elementarer Form oder in Form einer chemischen Verbindung mit dem Phosphitliganden umsetzt.

Als zusätzliche, im Reaktionsgemisch vorhandene Liganden können phosphorhaltiger Liganden, vorzugsweise Phosphine, Phosphite, Phosphonite oder Phosphinite eingesetzt werden.

Beispiele für solche Liganden sind:
Phosphine: Triphenylphosphin, Tris(p-tolyl)phosphin, Tris(m-tolyl)phosphin, Tris(o-tolyl)phosphin, Tris(p-methoxyphenyl)phosphin, Tris(p-dimethylaminophenyl)phosphin, Tricyclohexylphosphin, Tricyclopentylphosphin, Triethylphosphin, Tri-(1-naphthyl)phosphin, Tribenzylphosphin, Tri-n-butylphosphin, Tri-t-butylphosphin.
Phosphite: Trimethylphosphit, Triethylphosphit, Tri-n-propylphosphit, Tri-i-propylphosphit, Tri-n-butylphosphit, Tri-i-butylphosphit, Tri-t-butylphosphit, Tris(2-ethylhexyl)phosphit, Triphenylphosphit, Tris(2,4-di-t-butylphenyl)phosphit, Tris(2-t-butyl-4-methoxyphenyl)phosphit, Tris(2-*t*-butyl-4-methylphenyl)phosphit, Tris(p-kresyl)phosphit. Außerdem sind sterisch gehinderte Phosphitliganden, wie sie unter anderem in EP-A-155 508, US-A-4,668,651, US-A-4,748,261, US-A-4,769,498, US-A-4,774,361, US-A-4,835,299, US-A-4,885,401, US-A-5,059,710, US-A-5,113,022, US-A-5,179,055, US-A-5,260.491, US-A-5,264,616, US-A-5,288,918, US-A-5,360,938, EP-A-472 071, EP-A-518 241 und WO-A-97/20795 beschrieben werden, geeignete Liganden.
Phosphonite: Methyldiethoxyphosphin, Phenyldimethoxyphosphin, Phenyldiphenoxyphosphin, 2-Phenoxy-2H-dibenz[c,e][1,2]oxaphosphorin und dessen Derivate, in denen die Wasserstoffatome ganz oder teilweise durch Alkyl- und/oder Arylreste oder Halogenatome ersetzt sind und Liganden, die in WO-A-98/43935, JP-A-09-268152 und DE-A-198 10 794 und in den deutschen Patentanmeldungen DE-A-199 54 721 und DE-A-199 54 510 beschrieben sind.
Gängige Phosphinitliganden sind unter anderem in US-A-5,710,344, WO-A-95/06627, US-A-5,360,938 oder JP-A-07-082281 beschrieben. Beispiele hierfür sind Diphenyl(phenoxy)-phosphin und dessen Derivate, in denen die Wasserstoffatome ganz oder teilweise durch Alkyl- und/oder Arylreste oder Halogenatome ersetzt sind, Diphenyl(methoxy)phosphin und Diphenyl(ethoxy)phosphin.

Die erfindungsgemäßen Phosphite bzw. Phosphit-Rhodium-komplexe können in Verfahren zur Hydroformylierung von Olefinen, bevorzugt mit 2 bis 25 Kohlenstoffatomen, zu den entsprechenden Aldehyden eingesetzt werden.

Im Allgemeinen werden 1 bis 500 mol, vorzugsweise 1 bis 200 mol, bevorzugter 2 bis 50 mol des erfindungsgemäßen Phosphits pro mol Rhodium eingesetzt. Frischer Phosphitligand kann zu jedem Zeitpunkt der Reaktion zugesetzt werden, um die Konzentration an freiem Liganden konstant zu halten.

Die Konzentration des Rhodium im Reaktionsgemisch liegt im Bereich von 1 ppm bis 1000 ppm, vorzugsweise im Bereich von 5 ppm bis 300 ppm, bezogen auf das Gesamtgewicht der Reaktionsmischung.

Die mit den erfindungsgemäßen Phosphiten bzw. den entsprechenden Rhodium komplexen durchgeführten Hydroformylierungsreaktionen erfolgten nach bekannten Vorschriften, wie z. B. in J. FALBE, "New Syntheses with Carbon Monoxide", Springer Verlag, Berlin, Heidelberg, New York, Seite 95 ff., (1980) beschrieben. Die Olefinverbindung(en) wird (werden) in Gegenwart des Katalysators mit einem Gemisch aus CO und H₂ (Synthesegas) zu den um ein C-Atom reicheren Aldehyden umgesetzt.

Die Reaktionstemperaturen für ein Hydroformylierungsverfahren mit den erfindungsgemäßen Phosphiten bzw. Phosphit-Rhodium-komplexen als Katalysator liegen vorzugsweise zwischen 40 °C und 180°C und bevorzugter zwischen 75 °C und 140 °C. Die Drücke, unter denen die Hydroformylierung abläuft, betragen vorzugsweise 1 - 300 bar Synthesegas und bevorzugter 10 - 64 bar. Das Molverhältnis zwischen Wasserstoff und Kohlenmonoxid (H₂/CO) im Synthesegas beträgt vorzugsweise 10/1 bis 1/10 und bevorzugter 1/1 bis 2/1.
Der Katalysator bzw. der Ligand ist homogen im Hydroformylierungsgemisch, bestehend aus Edukten (Olefinen und Synthesegas) und Produkten (Aldehyden, Alkoholen, im Prozess gebildete Hochsieder), gelöst. Optional kann zusätzlich ein Lösungsmittel verwendet werden.

Aufgrund ihres hohen Molekulargewichtes besitzen die erfindungsgemäßen Phosphite eine geringe Flüchtigkeit. Sie können daher einfach von den leichter flüchtigen Reaktionsprodukten abgetrennt werden. Sie sind in den gängigen organischen Solventien ausreichend gut löslich.

Die Edukte für die Hydroformylierung sind Olefine oder Gemische von Olefinen mit 2 bis 25 Kohlenstoffatomen mit end- oder innenständiger C=C-Doppelbindung. Sie können geradkettig, verzweigt oder von cyclischer Struktur sein und auch mehrere olefinisch ungesättigte Gruppen aufweisen. Beispiele sind Propen; 1-Buten, cis-2-Buten, trans-2-Buten, Isobuten, Butadien, Mischungen der C₄-Olefine; C₅-Olefine wie 1-Penten, 2-Penten, 2-Methylbuten-1, 2-Methylbuten-2, 3-Methylbuten-1; C₆-Olefine wie 1-Hexen, 2-Hexen, 3-Hexen, das bei der Dimerisierung von Propen anfallende C₆-Olefingemisch (Dipropen); C₇-Olefine wie 1-Hepten, weitere n-Heptene, 2- Methyl-1-hexen, 3-Methyl-1-hexen; C₈-Olefine wie 1-Octen, weitere n-Octene, 2-Methylheptene, 3-Methylheptene, 5-Methylhepten-2, 6-Methylhepten-2, 2-Ethylhexen-1, das bei der Dimerisierung von Butenen anfallende isomere C₈-Olefingemisch (Dibuten); C₉-Olefine wie 1-Nonen, weitere n-Nonene, 2-Methyloctene, 3-Methyloctene, das bei der Trimerisierung von Propen anfallende C₉-Olefingemisch (Tripropen); C₁₀-Olefine wie n-Decene, 2-Ethyl-1-octen; C₁₂-Olefine wie n-Dodecene, das bei der Tetramerisierung von Propen oder der Trimerisierung von Butenen anfallende C₁₂-Olefingemisch (Tetrapropen oder Tributen), C₁₄-Olefine wie n-Tetradecene, C₁₆-Olefine wie n-Hexadecene, das bei der Tetramerisierung von Butenen anfallende C₁₆-Olefingemisch (Tetrabuten) sowie durch Cooligomerisierung von Olefinen mit unterschiedlicher C-Zahl (bevorzugt 2 bis 4) hergestellte Olefingemische, gegebenenfalls nach destillativer Trennung in Fraktionen mit gleicher oder ähnlicher C-Zahl. Ebenfalls können Olefine oder Olefingemische, die durch Fischer-Tropsch-Synthese erzeugt werden, eingesetzt werden, sowie Olefine, die durch Oligomerisierung von Ethen erhalten werden oder die über Methathesereaktionen oder Telomerisationsreaktion zugänglich sind.

Bevorzugte Edukte sind allgemein α-Olefine wie Propen, 1-Buten, 2-Buten, 1-Hexen, 1-Octen, sowie Dimere und Trimere des Butens (Dibuten, Di-n-buten, Di-iso-buten, Tributen).
Die Hydroformylierung kann kontinuierlich oder diskontinuierlich durchgeführt werden. Beispiele für technische Ausführungen sind Rührkessel, Blasensäulen, Strahldüsenreaktoren, Rohrreaktoren, oder Schlaufenreaktoren, die zum Teil kaskadiert und/oder mit Einbauten versehen sein können.

Die Reaktion kann durchgehend oder in mehreren Stufen erfolgen. Die Trennung der entstandenen Aldehydverbindungen und des Katalysators kann durch eine herkömmliche Methode, wie Fraktionierung, durchgeführt werden. Technisch kann dies beispielsweise über eine Destillation, über einen Fallfilmverdampfer oder einen Dünnschichtverdampfer erfolgen. Die gilt besonders, wenn der Katalysator in einem hochsiedenden Lösungsmittel gelöst von den niedriger siedenden Produkten abgetrennt wird. Die abgetrennte Katalysatorlösung kann für weitere Hydroformylierungen verwendet werden. Bei Einsatz niederer Olefine (z- B. Propen, Buten, Penten) ist auch ein Austrag der Produkte aus dem Reaktor über die Gasphase möglich.

Die folgenden Beispiele veranschaulichen die vorliegende Erfindung.

### Beispiele

Bei allen Beispielen wurde mit Standard-Schlenk-Technik unter Schutzgas gearbeitet. Die Lösungsmittel wurden vor Gebrauch über geeigneten Trocknungsmitteln getrocknet.

### Chlorverbindung A

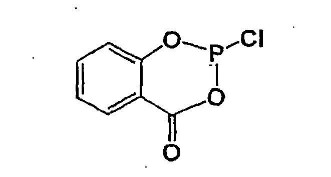

Chlorverbindung A (2-Chlor-4H-1,3,2-benzodioxaphosphorin-4-on) wurde von Fa. Aldrich, Taufkirchen, bezogen und verwendet wie geliefert.

### Chlorverbindung B

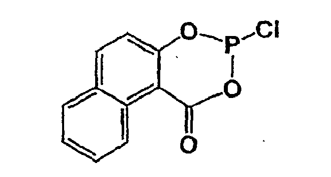

Die Darstellung der Chlorverbindung B erfolgte ausgehend von 2-Hydroxy-1-naphthalincarbonsäure in Anlehnung an BE 667036, Farbwerke Hoechst AG, 1966 ;Chem. Abstr. 65 (1966) 13741d. Die nachfolgende Synthesebeschreibung verdeutlicht das Vorgehen:
Umsetzung von 2-Hydroxy-1-naphthalincarbonsäure mit Phosphortrichlorid

In einem sekurierten 250 ml Schlenkrohr werden 9.22 g (0.049 mol) 2-Hydroxy-1-naphthalincarbonsäure, 200 ml getrocknetes Toluol und 0.48 g (0.005 mol) N-Methyl-2-pyrrolidinon vorgelegt. Zu dieser Mischung wird unter Rühren langsam 10.14 g (0.073 mol) Phosphortrichlorid zugegeben. Nach Anschluß des Schlenkrohres an eine Abgasleitung mit einem Gasdurchflussmesser wird die Reaktionsmischung vorsichtig auf 95 °C erhitzt und 5 h bei dieser Temperatur gehalten. Zur Aufarbeitung wird die Reaktionsmischung filtriert und das Lösemittel des Filtrats im Vakuum entfernt.
Ausbeute: 11.01 g (44.6 mmol), entsprechend 91.0 % der Theorie.
³¹P-NMR (Toluol-D₈) : δ 150.9 ppm

Herstellung von Calix[4]aren-bis-*O*-acylphosphit 1 (Diastereomerengemisch) 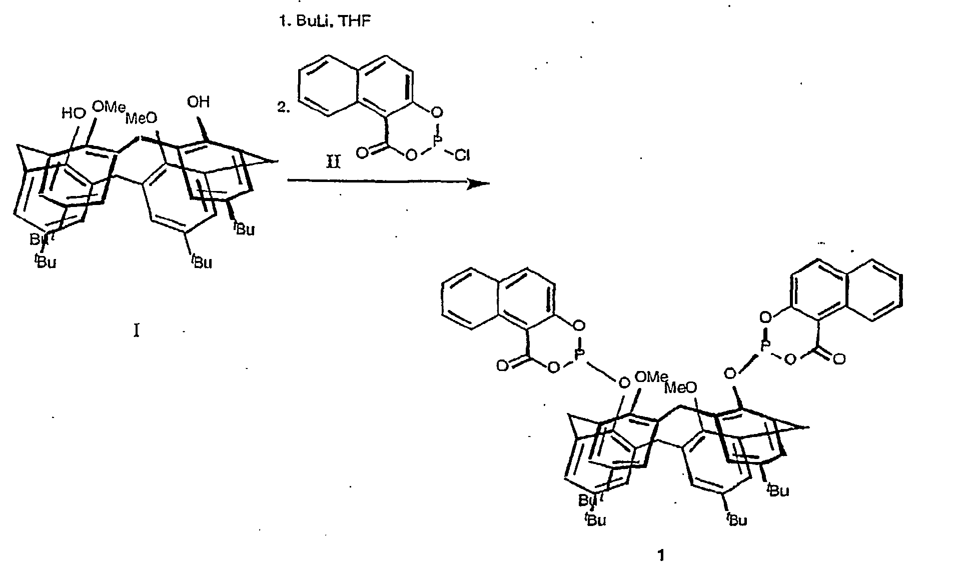 1.85 ml einer Lösung von n-Butyllithium (1.6 mol/l, 2.96 mmol) in Hexan wurde langsam bei Raumtemperatur zu einer Lösung von 1.0 g (1.48 mmol) *p*-*tert*-Butyl-bis-dimethoxycalix[4]aren I, gelöst in 40 ml THF gegeben. Die resultierende Lösung wurde 2 h bei RT gerührt. Anschließend wurden 0.75 g (2.96 mmol) von 3-Chlor-2,4-dioxa-3-phosphaphenanthren-1-on II, gelöst in 5 ml THF, über eine Kanüle hinzugetropft. Eine Farbänderung von farblos zu gelblich erfolgte nach vollendeter Zugabe. Die Lösung wurde über Nacht gerührt. Nach Abdampfen des Lösungsmittels erhielt man rohes Bisphosphit 1 als gelben Feststoff. Das Produkt wurde gereinigt durch Lösen in 30 ml CH₂Cl₂ und Filtration durch Celite^{®} Kieselgur-Filterhilfe. Nach Einengen der Lösung bei 50°C wurde 1 als gelblicher luft- und feuchtigkeitsempfindlicher Feststoff erhalten.
Ausbeute: 1.24 g (1.26 mol, 85 %);
m.p. 278°C.
¹H-NMR in CDCl₃ (400.1 MHz): □ = 0.51 (s, 18 H, C(CH₃)₃); 0.53 (s, 18 H, C(CH₃)₃); 0.92 (s, 18 H, C(CH₃)₃); 0.95 (s, 18 H, C(CH₃)₃); 3.10 (d, 4 H, ²J(HH) = 12.8 Hz, Ar-CH₂-Ar); 3.11 (d, 4 H, ²J(HH) = 13.0 Hz, Ar-CH₂-Ar); 3.68 (s, 6 H, O-CH₃); 3.73 (s, 6 H, O-CH₃); 4.26 (d, 4 H, ²J(HH) = 12.4 Hz, Ar-CH₂-Ar); 4.35 (d, 4 H, ²J(HH) = 12.8 Hz, Ar-CH₂-Ar); 6.11 (d, 2 H, ⁴J(HH) = 2.4 Hz, Ar-H); 6.13 (d, 2 H, ⁴J(HH) = 2.3 Hz, Ar-H); 6.38 (d, 2 H, ⁴J(HH) = 2.3 Hz, Ar-H); 6.42 (d, 2 H, ⁴J(HH) = 2.2 Hz, Ar-H); 6.55 (d, 2 H, ⁴J(HH) = 2.2 Hz, Ar-H); 6.62 (d, 2 H, ¹J(HH) = 2.3 Hz, Ar-H); 6.63 (d, 4 H, ⁴J(HH) = 2.3 Hz, Ar-H); 7.07 - 7.88 (m, 20 H, Naph-H); 8.96 (d, 2 H, ³J(HH) = 8.0 Hz, Naph-H); 8.99 (d, 2 H, ³J(HH) = 7.9 Hz, Naph-H).
¹³C-NMR in CDCl₃ (100.6 MHz): □ = 30.48 (s, 6 C, C(CH₃)₃); 30.66 (s, 6 C, C(CH₃)₃); 31.30 (s, 6 C, C(CH₃)₃); 31.33 (s, 6 C, C(CH₃)₃); 32.06 (s, 4 C, Ar-CH₂-Ar); 32.10 (s, 4 C, Ar-CH₂-Ar); 33.82 (s, 2 C, C(CH₃)₃); 33.84 (s, 2 C, C(CH₃)₃); 33.85 (s, 2 C, C(CH₃)₃); 33.87 (s, 2 C, C(CH₃)₃); 61.01 (s, 2 C, O-CH₃); 61.28 (s, 2 C, O-CH₃); 118.49 (s, 4 C, Naph-C); 124.79 - 129.50 (m, 40 C, Ar-C and Naph-C); 129.87 - 146.21 (m, 48 C, Ar-C_{quart.} and Naph-C_{quart.}). ³¹P-NMR in CDCl₃ (81.0 MHz): □ = 102.3, 103.5, 104.1, 104.6.
EI-MS, m/z (%): 1108 (4) [M]⁺; 892 (5) [M + H - R]⁺; 675 (50) [M - 2 R]⁺.

| Elementaranalyse: | | | | |
|---|---|---|---|---|
| C₆₈H₇₀O₁₀P₂ (1109.25) | ber.: | C 73.63 | H 6.36 | P 5.58 |
| | gef.: | C70.13 | H 6.37 | P 5.30 |

### Hydroformylierung von 1-Octen unter Verwendung von Calix[4]aren-bis-O-acylphosphit 1

Die Hydroformylierungen wurden in einem Buddeberg-200ml-Autoklaven mit Druckkonstanthaltung, Gasflußmessung und Begasungsrührer durchgeführt. Der Autoklav wurde unter Argonatmosphäre mit 10 ml einer 0.604 mM Lösung des Rhodiums in Form von [Rh(1,5-cyclooctadien)acac] (acac = Acetylacetonat-Anion) als Katalysatorvorstufe, 5 ml Toluol als GC-Standard und den entsprechenden Mengen an THF und Calix[4]aren-bis-*O*-acylphosphit 1 befüllt. In die Druckpipette wurden 15 ml 1-Octen eingespeist. Das Gesamtvolumen der Reaktionslösung betrug 56 ml. Nach Austausch der Argonatmosphäre durch Spülen mit Synthesegas (CO/H₂ 1:1) wurde das Rhodium-Ligand-Gemisch unter Rühren (1500 U/min) bei einem Synthesegasdruck von 30-33 bar auf 100 bzw. 120°C aufgeheizt. Bei Erreichen der gewünschten Reaktionstemperatur wurde der Synthesegasdruck auf 40 bzw. 50 bar erhöht und während der gesamten Reaktionsdauer über einen Druckregler konstant gehalten. Nach der Zugabe des Olefins wurde der Gasverbrauch mittels eines Hitec-Gasdurchflussmessers der Fa. Bronkhorst (NL) registriert. Die Reaktionsdauer der Hydroformylierungsversuche betrug jeweils 3 h. Anschließend wurde das Reaktionsgemisch auf Raumtemperatur abgekühlt, der Autoklav entspannt und mit Argon gespült. 2 ml der Autoklavenlösung wurden mit 10 ml Pentan versetzt und gaschromatographisch analysiert, wobei keine nachweisbare Olefinhydrierung und Alkoholbildung detektiert wurde.

**Table 1: Hydroformylierung von 1-Octen**

| | C₉-Aldehyde insgesamt, davon | geradkettiger C₉-Aldeyhd | verzweigte C₉-Aldehyde | | | |
|---|---|---|---|---|---|---|
| Molverhältnis Rh:Bisphosphit | Ausbeute [Mol%] | *n-*C9 [Mol%] | *i*-C8 [Mol%] | *i*-C7 [Mol%] | *i*-C6 [Mol%] | *n*/*iso* [Mol%] |
| 1:1 | 83.3 | 53.4 | 35.1 | 7.3 | 4.2 | 1.15 |
| 1:2 | 85.4 | 58.9 | 34.0 | 5.0 | 2.1 | 1.43 |
| 1:10 | 46.0 | 59.2 | 39.0 | 1.5 | 0.3 | 1.45 |

| | | | | | | |
|---|---|---|---|---|---|---|
| *n*-C9 = Nonanal *i*-C8 = 2-Methyloctanal *i*-C7 = 2-Ethylheptanal *i*-C6 = 2-Propylhexanal *n*/*iso* = Verhältnis von Nonanal/Summe aller verzweigten C₉-Aldehyde. | | | | | | |

## Patentansprüche

1. Phosphite gemäß Formel I oder II 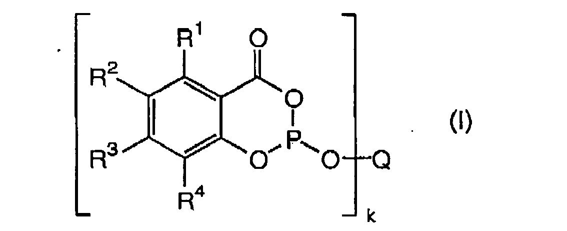 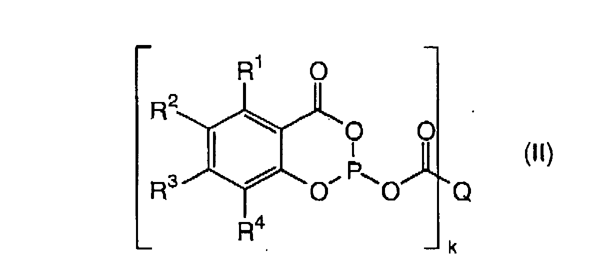 mit Ausnahme der Verbindung 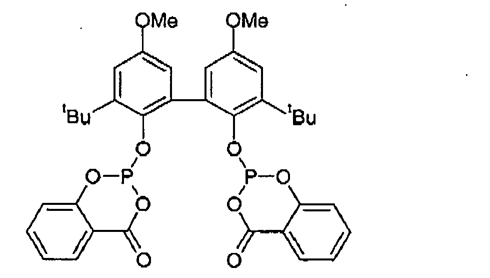 wobei R¹, R², R³, und R⁴ jeweils unabhängig voneinander ausgewählt sind aus einwertigen substituierten oder unsubstiuierten aliphatischen, alicyclischen, aromatischen, heteroaromatischen, gemischt aliphatisch-alicyclischen, gemischt aliphatisch-aromatischen, heterocyclischen, gemischt aliphatisch-heterocyclischen Kohlenwasserstoffresten mit 1 bis 50 Kohlenstoffatomen, H, F, Cl, Br, I, -CF₃, -CH₂(CF₂)ⱼCF₃ mit j = 0 - 9, -OR⁹, -COR⁹, -CO₂R⁹, -CO₂M, -SR⁹, -SO₂R⁹, -SOR⁹, -SO₃R⁹, -SO₃M, -SO₂NR⁹R¹⁰, -NR⁹R¹⁰, -N=CR⁹R¹⁰, wobei R⁹ und R¹⁰ unabhängig voneinander aus H, einwertigen substituierten oder unsubstituierten aliphatischen und aromatischen Kohlenwasserstoffresten mit 1 bis 25 Kohlenstoffatomen ausgewählt sind und M ein Alkalimetall-, formal ein halbes Erdalkalimetall-, Ammonium- oder Phosphoniumion ist,
oder benachbarte Reste R¹ bis R⁴ zusammen ein kondensiertes substituiertes oder unsubstituiertes aromatisches, heteroaromatisches, aliphatisches, gemischt aromatisch-aliphatisches oder gemischt heteroaromatisch-aliphatisches Ringsystem ausbilden;
k mindestens 2 ist;
Q ein zweibindiger Kohlenwasserstoffrest gemäß Formel III 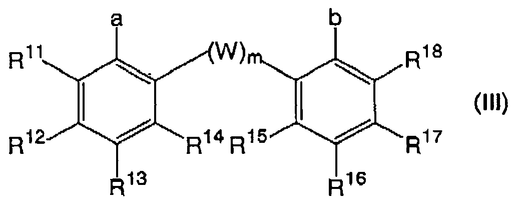 ist und R¹¹, R¹², R¹³, R¹⁴, R¹⁵, R¹⁶, R¹⁷ und R¹⁸ wie R¹ bis R⁴ definiert sind, W ein zweiwertiger Rest CR¹⁹R²⁰, wobei R¹⁹ und R²⁰ wie R⁹ und R¹⁰ definiert sind, m = 0 - 1 und die Positionen a und b die Anknüpfpunkte darstellen, oder
Q ein k-bindiger Calix[n]arenrest gemäß Formel IV 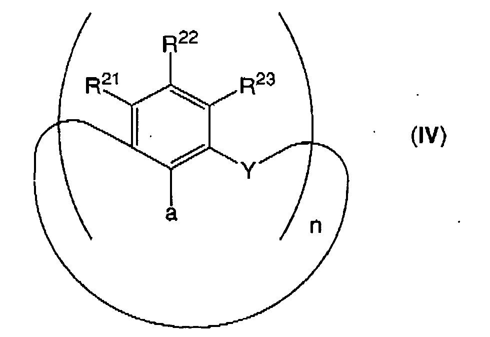 ist, wobei R²¹, R²², und R²³ unabhängig voneinander ausgewählt sind aus substituierten oder unsubstituierten aliphatischen und aromatischen Kohlexiwasserstoffresten mit 1 bis 25 Kohlenstoffatomen, H, F, Cl, Br, I, -CF₃, -CH₂(CF₂)ⱼCF₃ mit j = 0 - 9, -OR²⁴, -COR²⁴, -CO₂R²⁴, -CO₂M, -SR²⁴, -SO₂R²⁴, -SOR²⁴, -SO₃R²⁴, -SO₃M, -SO₂NR²⁴R²⁵, -NR²⁴R²⁵, -N=CR²⁴R²⁵, wobei R²⁴ und R²⁵ wie R⁹ und R¹⁰ definiert sind und M ein Alkalimetall-, formal ein halbes Erdalkalimetall-, Ammonium- oder Phosphoniumion ist,
oder benachbarte Reste R²¹ bis R²³ zusammen ein kondensiertes substituiertes oder unsubstituiertes aromatisches, heteroaromatisches, aliphatisches, gemischt aromatisch-aliphatisches oder gemischt heteroaromatisch-aliphatisches Ringsystem ausbilden;
Y ein zweiwertiger Rest CR²⁴R²⁵ oder CR²⁴R²⁵-O-CR²⁴R²⁵ ist, n = 3 - 6 ist und die Positionen a die Anknüpfpunkte darstellen oder für OR²⁴ stehen, wobei R²⁴ und R²⁵ wie oben definiert sind.

2. Phosphit nach Anspruch 1, wobei mindestens zwei benachbarte Reste R¹ bis R⁴ zusammen ein kondensiertes aromatisches, heteroaromatisches, aliphatisches, gemischt aromatisch-aliphatisches oder gemischt heteroaroznatxsch-aliphatisches Ringsystem ausbilden, das unsubstituiert ist oder mit mindestens einem Rest, ausgewählt aus aliphatischen, alicyclischen, aromatischen, heteroaromatischen, gemischt aliphatisch-alicyclischen, gemischt aliphatisch-aromatischen, heterocyclischen, gemischt aliphatisch-heterocyclischen Kohlenwasserstoffresten mit 1 bis 50 Kohlenstoffatomen, F, Cl, Br, I, -CF₃, -CH₂(CF₂)ⱼCF₃ mit j = 0 - 9, -OR⁹, -COR⁹, -CO₂R⁹, -CO₂M, -SR⁹, -SO₂R⁹, -SOR⁹, -SO₃R⁹, -SO₃M, -SO₂NR⁹R¹⁰, -NR⁹R¹⁰, oder -N=CR⁹R¹⁰, substituiert ist, wobei R⁹, R¹⁰ und M wie in Anspruch 1 definiert sind.

3. Phosphitmetallkomplex, enthaltend Rhodium und ein oder mehrere Phosphite gemäß einem der Ansprüche 1 oder 2.

4. Verwendung eines Phosphits gemäß einem der Ansprüche 1 oder 2 oder eines Phosphitmetallkomplexes nach Anspruch 3 in einem Verfahren zur Hydroformylierung von Olefinen.

5. Verwendung nach Anspruch 4, wobei weitere phosphorhaltige Liganden anwesend sind.

6. Verfahren zur Hydroformylierung von Olefinen, umfassend die Umsetzung eines Monoolefins oder Monoolefingemisches mit einem Gemisch aus Kohlenmonoxid und Wasserstoff in Gegenwart eines Phosphitmetallkomplex nach Anspruch 3.

7. Verfahren zur Herstellung eines Phosphits nach einem der Ansprüche 1 oder 2, umfassend
(a) Umsetzung einer α-Hydroxyarylcarbonsäure gemäß Formel (1) 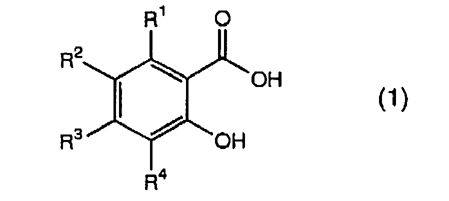 mit PCl₃, PBr₃ oder PI₃ in Gegenwart einer Base unter Ausbildung eines Halogendioxaphosphorinons gemäß Formel (2), wobei Hal = Cl, Br oder I, und 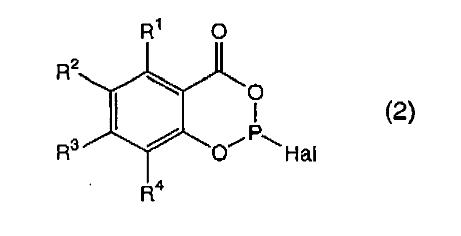
(b) Umsetzung des Halogendioxaphosphorinons (2) in Gegenwart einer Base mit
(i) einem Diol HO-Q-OH, um ein Phosphit gemäß Formel (I) zu erhalten, oder
(ii) einer Dicarbonsäure HOOC-Q-COOH, um ein Phosphit gemäß Formel (II) zu erhalten,
wobei R¹ bis R⁴ und Q wie in Anspruch 1 definiert sind.

8. Verfahren zur Herstellung eines Phosphits nach Anspruch 7, wobei in Schritt (a) zwei unterschiedlich substituierte Halogendioxaphosphorinone (2a) und (2b) synthetisiert werden, die dann in Schritt (b)(i) nacheinander mit einem Diol oder in Schritt (b)(ii) nacheinander mit einer Dicarbonsäure umgesetzt werden, um ein unsymmetrisches Phosphit zu erhalten.

9. Verfahren zur Herstellung eines Phosphitmetallkomplexes nach Anspruch 3, umfassend die Umsetzung von Rhodium in elementarer Form oder in Form einer chemischen Verbindung mit einem Phosphit nach einem der Ansprüche 1 oder 2.

## Claims

1. Phosphites of the formula I or II, 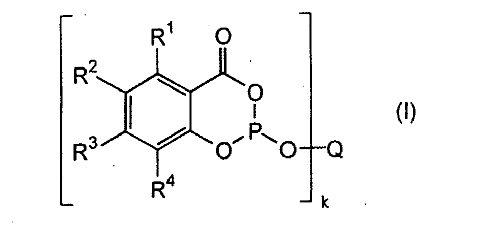 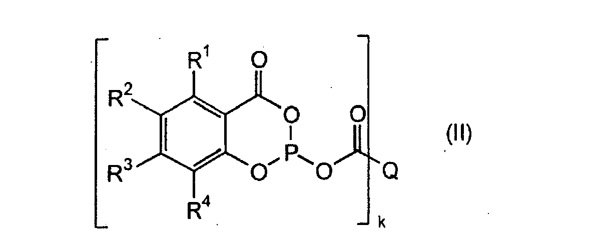 with the exception of the compound 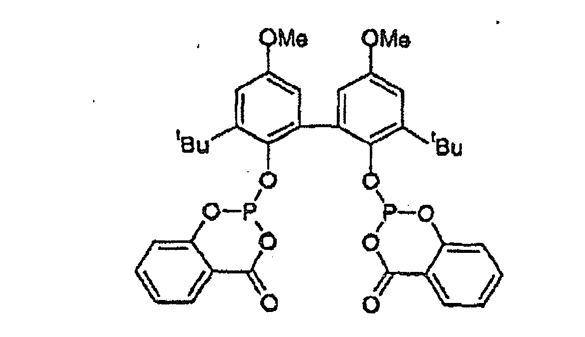 where R¹, R², R³ and R⁴ are each selected independently from among monovalent substituted or unsubstituted aliphatic, alicyclic, aromatic, heteroaromatic, mixed aliphatic-alicyclic, mixed aliphatic-aromatic, heterocyclic, mixed aliphatic-heterocyclic hydrocarbon radicals having from 1 to 50 carbon atoms, H, F, Cl, Br, I, -CF₃, -CH₂(CF₂)ⱼCF₃ where j = 0 - 9, -OR⁹, -COR⁹, CO₂R⁹, -CO₂M, -SR⁹, -SO₂R⁹, -SOR⁹, -SO₃R⁹, -SO₃M, -SO₂NR⁹R¹⁰, -NR⁹R¹⁰, -N=CR⁹R¹⁰, where R⁹ and R¹⁰ are selected independently from among H, monovalent substituted or unsubstituted aliphatic and aromatic hydrocarbon radicals having from 1 to 25 carbon atoms and M is an alkali metal ion, formally half an alkaline earth metal ion, an ammonium ion or phosphonium ion,
or adjacent radicals R¹ to R⁴ together form a fused substituted or unsubstituted aromatic, heteroaromatic, aliphatic, mixed aromatic-aliphatic or mixed heteroaromatic-aliphatic ring system;
k is at least 2;
Q is a divalent hydrocarbon radical of the formula III 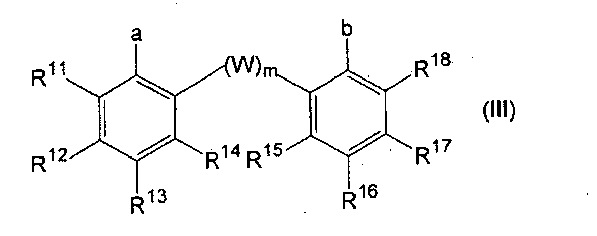 and R¹¹, R¹², R¹³, R¹⁴, R¹⁵, R¹⁶, R¹⁷ and R¹⁸ are as defined for R¹ to R⁴, W is a divalent radical CR¹⁹R²⁰, where R¹⁹ and R²⁰ are as defined for R⁹ and R¹⁰, m = 0 -1 and the positions a and b represent the linkage points, or Q is a k-valent calix[n]arene radical of the formula IV 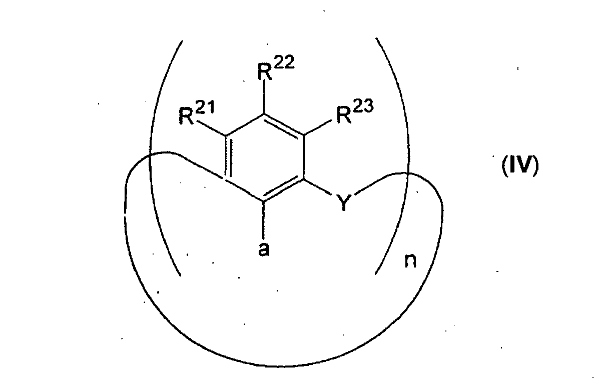 where R²¹, R²² and R²³ are selected independently from among substituted or unsubstituted aliphatic and aromatic hydrocarbon radicals having from 1 to 25 carbon atoms, H, F, Cl, Br, I, -CF₃, -CH₂(CF₂)ⱼCF₃ where j = 0 - 9, -OR²⁴, -COR²⁴, -CO₂R²⁴, -CO₂M, -SR²⁴, -SO₂R²⁴, -SOR²⁴, -SO₃R²⁴, -SO₃M, -SO₂NR²⁴R²⁵, -NR²⁴R²⁵, -N=CR²⁴R²⁵, where R²⁴ and R²⁵ are as defined for R⁹ and R¹⁰ and M is an alkali metal ion, formally half an alkaline earth metal ion, an ammonium ion or phosphonium ion,
or adjacent radicals R²¹ to R²³ together form a fused substituted or unsubstituted aromatic, heteroaromatic, aliphatic, mixed aromatic-aliphatic or mixed heteroaromatic-aliphatic ring system;
Y is a divalent radical CR²⁴R²⁵ or CR²⁴R²⁵-O-CR²⁴R²⁵,
n = 3 - 6 and the positions a represent the linkage points or are occupied by OR²⁴, where R²⁴ and R²⁵ are as defined above.

2. Phosphite according to Claim 1, wherein at least two adjacent radicals R¹ to R⁴ together form a fused aromatic, heteroaromatic, aliphatic, mixed aromatic-aliphatic or mixed heteroaromatic-aliphatic ring system which is unsubstituted or is substituted by at least one radical selected from among aliphatic, alicyclic, aromatic, heteroaromatic, mixed aliphatic-alicyclic, mixed aliphatic-aromatic, heterocyclic, mixed aliphatic-heterocyclic hydrocarbon radicals having from 1 to 50 carbon atoms, F, Cl, Br, I, -CF₃, -CH₂(CF₂)ⱼCF₃ where j = 0 - 9, -OR⁹, COR⁹, CO₂R⁹, -CO₂M, -SR⁹, -SO₂R⁹, SOR⁹, SO₃R⁹, -SO₃M, -SO₂NR⁹R¹⁰, -NR⁹R¹⁰ or -N=CR⁹R¹⁰, where R⁹, R¹⁰ and M are as defined in Claim 1.

3. Phosphite-metal complex comprising rhodium and one or more phosphites according to either of Claims 1 and 2.

4. Use of a phosphite according to either of Claims 1 and 2 or of a phosphite-metal complex according to Claim 3 in a process for the hydroformylation of olefins.

5. Use according to Claim 4, wherein further phosphorus-containing ligands are present.

6. Process for the hydroformylation of olefins, which comprises reacting a monoolefin or monoolefin mixture with a mixture of carbon monoxide and hydrogen in the presence of a phosphite-metal complex according to Claim 3.

7. Process for preparing a phosphite according to either of Claims 1 and 2, which comprises
(a) reacting an α-hydroxyarylcarboxylic acid of the formula (1) 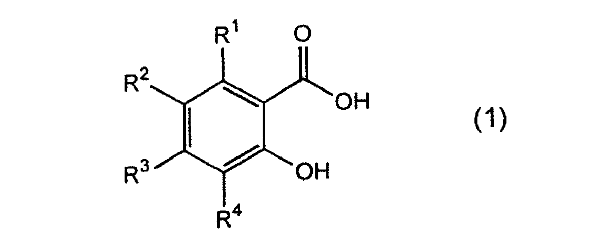 with PCl₃, PBr₃ or PI₃ in the presence of a base to form a halodioxaphosphorinone of the formula (2), 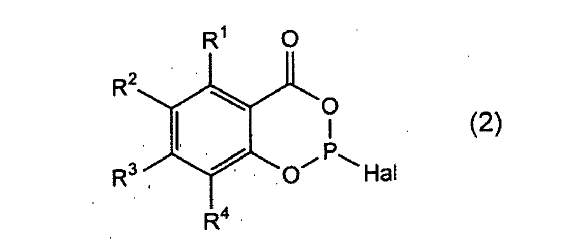 where Hal = Cl, Br or I, and
(b) reacting the halodioxaphosphorinone (2) in the presence of a base with
(i) a diol HO-Q-OH to give a phosphite of the formula (I), or
(ii) a dicarboxylic acid HOOC-Q-COOH to give a phosphite of the formula (II),
where R¹ to R⁴ and Q are as defined in Claim 1.

8. Process for preparing a phosphite according to Claim 7, wherein two differently substituted halodioxaphosphorinones (2a) and (2b) are synthesized in step (a) and are then reacted in succession with a diol in step (b)(i) or reacted in succession with a dicarboxylic acid in step (b) (ii) to give an unsymmetrical phosphite.

9. Process for preparing a phosphite-metal complex according to Claim 3, which comprises reacting rhodium in elemental form or in the form of a chemical compound with a phosphite according to either of Claims 1 and 2.

## Revendications

1. Phosphites de formule I ou II : 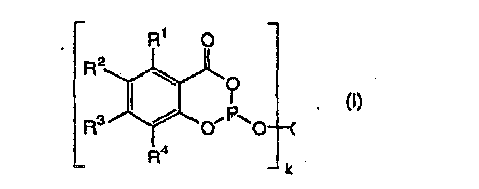 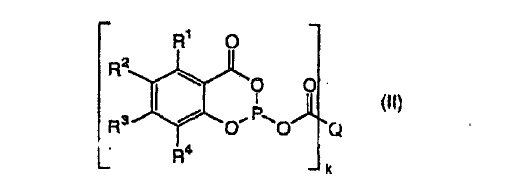 à l'exception du composé : 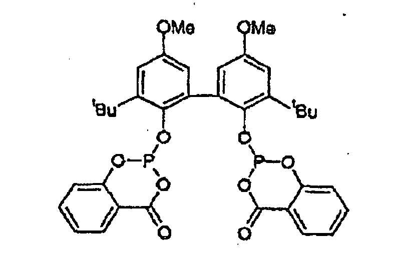 formules dans lesquelles R¹, R², R³ et R⁴ sont à chaque fois choisis indépendamment les uns des autres parmi des résidus hydrocarbures aliphatiques, alicycliques, aromatiques, hétéroaromatiques, mixtes aliphatiques/alicycliques, mixtes aliphatiques/aromatiques, hétérocycliques, mixtes aliphatiques/hétérocycliques monovalents, substitués ou non substitués, de 1 à 50 atomes de carbone, H, F, Cl, Br, I, -CF₃, -CH₂(CF₂)ⱼCF₃ avec j = 0 à 9, -OR⁹, -COR⁹, -CO₂R⁹, -CO₂M, -SR⁹, -SO₂R⁹,-SOR⁹, -SO₃R⁹, -SO₃M, -SO₂NR⁹R¹⁰, -NR⁹R¹⁰, -N=CR⁹R¹⁰, où R⁹ et R¹⁰ sont choisis indépendamment l'un de l'autre parmi H, des résidus hydrocarbures aliphatiques et aromatiques monovalents, substitués ou non substitués, de 1 à 25 atomes de carbone, et M est un ion de métal alcalin, d'un point de vue formel un demi-ion de métal alcalino-terreux, un ion d'ammonium ou un ion de phosphonium,
ou bien des résidus R¹ à R⁴ voisins pris ensemble forment un système cyclique aromatique, hétéroaromatique, aliphatique, mixte aromatique/aliphatique ou mixte hétéroaromatique/aliphatique condensé, substitué ou non substitué ;
k est au moins égal à 2 ;
Q est un résidu hydrocarbure à deux liaisons de formule III : 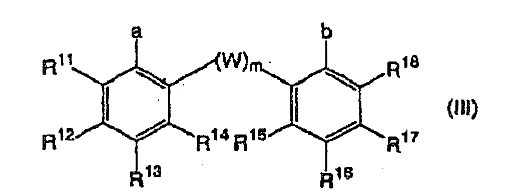 et R¹¹, R¹², R¹³, R¹⁴, R¹⁵, R¹⁶, R¹⁷ et R¹⁸ ont les mêmes définitions que R¹ à R⁴, W est un résidu divalent CR¹⁹R²⁰, où R¹⁹ et R²⁰ ont les mêmes définitions que R⁹ et R¹⁰, m = 0 à 1, et les positions a et b désignent les points de rattachement, ou
Q est un résidu calix[n]arène à k liaisons de formule IV : 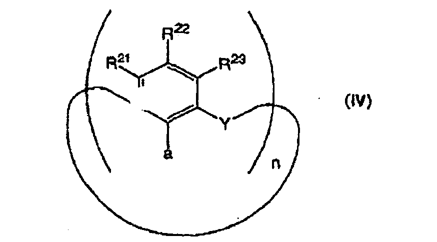 dans laquelle R²¹, R²² et R²³ sont choisis indépendamment les uns des autres parmi des résidus hydrocarbures aliphatiques et aromatiques, substitués ou non substitués, de 1 à 25 atomes de carbone, H, F, Cl, Br, I, -CF₃, -CH₂(CF₂)ⱼCF₃ avec j = 0 à 9, -OR²⁴, -COR²⁴, -CO₂R²⁴, -CO₂M, -SR²⁴, -SO₂R²⁴, -SOR²⁴, -SO₃R²⁴, -SO₃M, -SO₂NR²⁴R²⁵, -NR²⁴R²⁵, -N=CR²⁴R²⁵, où R²⁴ et R²⁵ ont les mêmes définitions que R⁹ et R¹⁰, et M est un ion de métal alcalin, d'un point de vue formel un demi-ion de métal alcalino-terreux, un ion d'ammonium ou un ion de phosphonium,
ou bien des résidus R²¹ à R²³ voisins pris ensemble forment un système cyclique aromatique, hétéroaromatique, aliphatique, mixte aromatique/aliphatique ou mixte hétéroaromatique/aliphatique condensé, substitué ou non substitué ;
Y est un résidu divalent CR²⁴R²⁵ ou CR²⁴R²⁵-O-CR²⁴R²⁵, n = 3 à 6, et les positions a désignent les points de rattachement ou bien représentent OR²⁴, où R²⁴ et R²⁵ sont tels que définis ci-dessus.

2. Phosphite selon la revendication 1, dans lequel au moins deux résidus R¹ à R⁴ voisins pris ensemble forment un système cyclique aromatique, hétéroaromatique, aliphatique, mixte aromatique/aliphatique ou mixte hétéroaromatique/aliphatique condensé qui est non substitué ou substitué par au moins un résidu choisi parmi des résidus hydrocarbures aliphatiques, alicycliques, aromatiques, hétéroaromatiques, mixtes aliphatiques/alicycliques, mixtes aliphatiques/aromatiques, hétérocycliques, mixtes aliphatiques/hétérocycliques de 1 à 50 atomes de carbone, F, Cl, Br, I, -CF₃, -CH₂(CF₂)ⱼCF₃ avec j = 0 à 9, -OR⁹, -COR⁹, -CO₂R⁹, -CO₂M, -SR⁹, -SO₂R⁹, -SOR⁹, -SO₃R⁹, -SO₃M, -SO₂NR⁹R¹⁰, -NR⁹R¹⁰ ou -N=CR⁹R¹⁰, où R⁹, R¹⁰ et M sont tels que définis dans la revendication 1.

3. Complexe phosphite/métal, contenant du rhodium et un ou plusieurs phosphites selon la revendication 1 ou la revendication 2.

4. Utilisation d'un phosphite selon la revendication 1 ou la revendication 2 ou d'un complexe phosphite/métal selon la revendication 3 dans un procédé d'hydroformylation d'oléfines.

5. Utilisation selon la revendication 4, dans laquelle d'autres ligands contenant du phosphore sont présents.

6. Procédé d'hydroformylation d'oléfines, comprenant la réaction d'une mono-oléfine ou d'un mélange de mono-oléfines avec un mélange de monoxyde de carbone et d'hydrogène en présence d'un complexe phosphite/ métal selon la revendication 3.

7. Procédé de production d'un phosphite selon la revendication 1 ou la revendication 2, comprenant :
(a) la réaction d'un acide α-hydroxyarylcarboxylique de formule (1) : 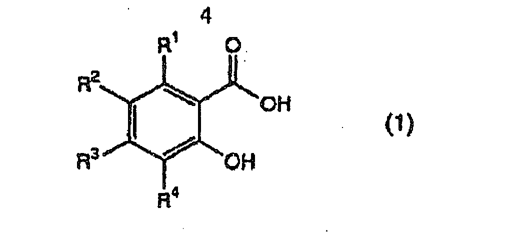 avec du PCl₃, du PBr₃ ou du PI₃ en présence d'une base avec formation d'une halodioxaphosphorinone de formule (2) dans laquelle Hal représente Cl, Br ou I, et : 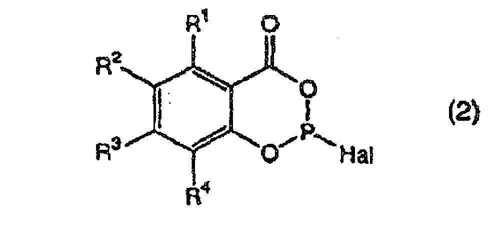
(b) la réaction de la halodioxaphosphorinone (2) en présence d'une base avec :
(i) un diol HO-Q-OH pour obtenir un phosphite de formule (I), ou
(ii) un acide dicarboxylique HOOC-Q-COOH pour obtenir un phosphite de formule (II),
R¹ à R⁴ et Q ayant les définitions indiquées dans la revendication 1.

8. Procédé de production d'un phosphite selon la revendication 7, dans lequel, à l'étape (a), deux halodioxaphosphorinones différemment substituées (2a) et,(2b) sont synthétisées et ensuite mises à réagir l'une après l'autre avec un diol à l'étape (b)(i) ou avec un acide dicarboxylique à l'étape (b)(ii) afin d'obtenir un phosphite asymétrique.

9. Procédé de production d'un complexe phosphite/métal selon la revendication 3, comprenant la réaction de rhodium sous forme élémentaire ou sous la forme d'un composé chimique avec un phosphite selon la revendication 1 ou la revendication 2.
